# FASCICULE DE BREVET EUROPEEN

(11) **EP 1 945 341 B1**
(45) Date de publication et mention de la délivrance du brevet: **23.12.2009**
(21) Numéro de dépôt: 06831040.8
(22) Date de dépôt: 31.10.2006
(51) Int. Cl.: B01J 2/30, A61K 9/14, A61K 9/16, A23P 1/06

(54) **COMPOSITIONS SECHES STABILISEES, LEURS PROCEDES D'OBTENTION ET LEURS UTILISATIONS**
STABILISIERTE TROCKENZUSAMMENSETZUNGEN, VERFAHREN ZU IHRER GEWINNUNG UND VERWENDUNG
STABILIZED DRY COMPOSITIONS, METHOD FOR OBTAINING THEM AND USE THEREOF

(30) Priorité: 02.11.2005 FR 0511148
(43) Date de publication de la demande: 23.07.2008
(73) Titulaire: VIRBAC S.A., F-06516 Carros (FR)
(72) Inventeur: DERRIEU, Guy, F-06800 Cagnes sur Mer (FR)
(74) Mandataire: Macquet, Christophe
(86) Numéro de dépôt international: PCT/FR2006/002428
(87) Numéro de publication internationale: WO 2007/051918

(56) Documents cités:
- DE-A1- 2 217 386
- FR-A- 2 863 914
- GB-A- 973 293
- GB-A- 1 168 106
- US-A- 3 367 883

## Description

L'invention concerne des compositions sèches stabilisées comprenant au moins un principe actif, leurs procédés d'obtention, ainsi que leurs utilisations.

Les produits pulvérulents et, plus particulièrement, les compositions alimentaires ou les médicaments, présentés sous forme sèche en poudre, sont connus pour prendre en masse. Ce changement d'état physique, outre le fait d'empêcher la commercialisation et l'utilisation du produit, conduit à des dégradations chimiques qui nuisent à l'efficacité de la composition et peuvent également être dangereuses lors de son utilisation. Cette prise en masse, appelée également mottage, est principalement induite par la présence d'eau.

La présence de cette eau est due à plusieurs facteurs. Tout d'abord, elle est due à la nature de l'emballage qui, selon sa structure intrinsèque, présente une certaine porosité aux gaz et, par suite, à la vapeur d'eau. Ensuite, elle est due à la présence même infime, dans la composition, d'eau liée à la structure moléculaire, d'eau de cristallisation et/ou d'eau d'absorption ou humidité relative.

Bien entendu, le mottage est très fortement influencé par des facteurs extérieurs comme la température ou l'humidité et, plus certainement, par leur combinaison.

On a cherché à développer de nouveaux moyens pour lutter contre le phénomène de mottage, notamment en développant des procédés d'enrobage ou de granulation de poudre, en ajoutant, à la composition, des poudres poreuses ou absorbant l'eau telle que la poudre d'amidon ou la poudre d'anhydride silicique, ou alors, par la mise au point d'emballages de plus en plus performants.

Si la mise en oeuvre de procédés d'enrobage et de granulation semble répondre à la problématique de la présente invention, ces opérations s'avèrent être relativement complexes, longues et onéreuses. En outre, chaque application constitue un cas particulier. Ainsi, l'adjuvant d'enrobage ou de granulation est spécifique de la composition, de même que le procédé de fabrication. En pratique, cela est nécessaire pour éviter d'engendrer toutes actions négatives pour les actifs de la composition. Par ailleurs, l'adjonction de poudre poreuse ou absorbant l'eau est largement utilisée mais elle montre vite des limites en n'empêchant pas la prise en masse des compositions sèches pulvérulentes.

On se référera notamment aux documents de l'état de la technique suivants. Tout d'abord, le document brevet EP 1 382 245 divulgue notamment une méthode d'anti-mottage pour des principes actifs agricoles par ajout d'une substance inorganique de type silice. Ensuite, le document brevet US 2003/0074940, décrit une combinaison d'adjuvants composée de granules d'engrais enrobés par un agent réducteur et éventuellement un agent antimottant.

On notera enfin, l'enseignement du document brevet US 2005/0003043, qui divulgue une méthode d'anti-mottage de produits protéiques. Cette méthode consiste à ajouter au moins un agent réducteur et un chaotrope, composé qui dénature les protéines, à la composition protéique. Toutefois, la déshydratation et la dénaturation de composés peut entraîner une accélération de leur dégradation mais aussi modifier leur solubilité.

Le document US-A-3 367 883 décrit une composition sèche stabilisée constituée d'une partie pulvérulente avec des particules antimottantes de dimensions inférieures à la partie pulvérulente de base.

Compte tenu de ce qui précède, un problème que se propose de résoudre l'invention est de réaliser des compositions sèches stabilisées, comprenant au moins un principe actif, qui ne présentent pas de prise en masse lors de leur conservation.

La solution de l'invention à ce problème posé a pour premier objet une composition sèche stabilisée, comprenant au moins un principe actif, constituée d'une partie pulvérulente de granulométrie moyenne comprise entre 1 et 250 µm, **caractérisée en ce qu**'elle incorpore des particules antimottantes dont la granulométrie moyenne est comprise entre 200 et 1500 µm et est au moins 2,5 fois supérieure à la granulométrie moyenne de ladite partie pulvérulente.

Elle a pour second objet un procédé de fabrication d'une composition sèche stabilisée, comprenant au moins un principe actif, constituée d'une partie pulvérulente de granulométrie moyenne comprise entre 1 et 250 µm, **caractérisé en ce qu**'il comporte l'étape de mélange de ladite partie pulvérulente avec des particules antimottantes dont la granulométrie moyenne est comprise entre 200 et 1500 µm et est au moins 2,5 fois supérieure à la granulométrie moyenne de ladite partie pulvérulente.

Enfin, elle a pour troisième objet l'utilisation d'une composition telle que ci-dessus définie, comme aliment, complément alimentaire ou médicament conditionné sous différentes formes.

De manière surprenante, lorsqu'une composition comprend à la fois une partie pulvérulente de granulométrie comprise entre 1 et 250 µm et des particules antimottantes dont la granulométrie moyenne est comprise entre 200 et 1500 µm et est au moins 2,5 fois supérieure à la granulométrie moyenne de la partie pulvérulente, elle ne prend pas en masse et ne subit aucune modification physique ou chimique.

L'invention sera mieux comprise à la lecture de la description non limitative qui va suivre.

La composition sèche stabilisée selon l'invention comprend au moins un principe actif et est constituée d'une partie pulvérulente et de particules antimottantes qui se distinguent principalement par leur granulométrie.

La partie pulvérulente selon l'invention se présente sous la forme d'une poudre fine composée de particules sèches, au moins à leur surface. De façon avantageuse, ladite poudre possède une granulométrie moyenne comprise entre 1 et 250 µm et, plus préférentiellement, entre 10 et 100 µm.

Selon l'invention, la partie pulvérulente de la composition est présente dans des concentrations allant de préférence de 40% à 95% en poids par rapport au poids total de la composition.

La partie pulvérulente est constituée de principes actifs, d'excipients ou d'un mélange des deux. De manière avantageuse, dans le cadre d'un mélange, la granulométrie moyenne des principes actifs et des excipients sera approximativement identique.

L'excipient ou le mélange d'excipients susceptibles d'entrer dans la formulation de la partie pulvérulente de la composition selon l'invention sont choisis parmi les sucres tels que le lactose, le saccharose, le dextrose ou les maltodextrines, les sels inorganiques tels que le phosphate dicalcique ou le carbonate de calcium, les acides organiques et leurs sels tels que l'acide citrique, l'acétate de sodium ou le propionate de potassium, les farines végétales et leurs sous-produits tels que la farine de blé, le son, l'amidon de riz ou le gluten maïs, ou leur mélange.

Selon un mode de réalisation avantageux de l'invention, la partie pulvérulente est uniquement constituée de principes actifs.

Selon l'invention, les particules antimottantes de l'invention sont également sèches, au moins à leur surface, et se présentent bien souvent sous une forme sphérique, ovoïde ou allongée. Leur surface est lisse, poreuse et/ou creuse et ce, de manière régulière ou irrégulière. De façon avantageuse, les particules antimottantes présentent une granulométrie allant de 200 à 1500 µm, plus préférentiellement de 250 à 800 µm, et encore plus préférentiellement de 300 à 700 µm.

Les particules antimottantes de la composition selon l'invention sont constituées de principes actifs, d'excipients ou d'un mélange des deux. Préférentiellement, les principes actifs sont compris, partiellement ou en totalité, dans les particules antimottantes de la composition selon l'invention.

De manière préférée, l'excipient ou le mélange d'excipients susceptibles d'entrer dans la composition des particules antimottantes de la composition selon l'invention sont identiques à l'excipient ou au mélange d'excipients entrant dans la composition de la partie pulvérulente.

Selon un mode de réalisation avantageux de l'invention, les particules antimottantes de la composition selon l'invention sont des corps simples ou complexes utilisés dans la fabrication ou la préparation d'une composition alimentaire ou médicamenteuse. Il s'agit en particulier de particules de sucres, de mélanges de saccharides, d'acides organiques et leurs sels, de polyols, de sels inorganiques ou de particules creuses à base d'au moins un polymère ou copolymère acrylique ou méthacrylique, pris seuls ou en mélange. A titre d'exemple non limitatif dé sucres, on choisira avantageusement le lactose ou le saccharose. A titre d'exemple non limitatif de mélanges de saccharides, on choisira avantageusement les dextrates. A titre d'exemple non limitatif d'acides organiques, on choisira avantageusement l'acide citrique. A titre d'exemple non limitatif de polyols, on choisira avantageusement le mannitol ou le sorbitol. A titre d'exemple non limitatif de sels inorganiques, on choisira avantageusement le phosphate de calcium hydraté. A titre d'exemple non limitatif de particules creuses à base d'au moins un polymère ou copolymère acrylique ou méthacrylique, on choisira avantageusement les particules de polyméthacrylate.

De telles particules antimottantes peuvent avantageusement être obtenues par granulation et par suite se présenter sous la forme de granulés. Une telle granulation peut être effectuée par mélange en milieu humide, et comprendre des étapes de séchage puis de calibrage. Elle peut aussi être réalisée en lit d'air fluidisé ou sur des appareils connus de l'état de la technique combinant un ensemble d'opérations menant aux particules souhaitées, par compactage, par granulation ou par enrobage.

Selon l'invention, les particules antimottantes sont présentes dans des concentrations allant de préférence de 5 à 60% en poids par rapport au poids total de la composition et, plus préférentiellement, de 10 à 40%.

Le principe actif selon l'invention est défini comme tout corps simple ou complexe ayant une activité de n'importe quelle nature sur son environnement. Il vient par définition s'opposer à un excipient qui, simple ou complexe, n'a aucune activité sur l'environnement ou, sur le ou les principes actifs.

Les principes actifs selon l'invention, présents dans la partie pulvérulente et/ou dans les particules antimottantes, sont des corps simples ou complexes choisis parmi les vitamines, les minéraux, lés oligo-éléments, les acides aminés, les protéines, les enzymes, les facteurs de croissance, les poudres de plantes ou leurs extraits, les inhibiteurs de croissance des insectes, les anti-bactériens, les antifongiques, les antiseptiques, les antibiotiques, les antibiomimétiques, les antiparasitaires et/ou les anti-infectieux de synthèse, pris seuls ou en mélange.

Le choix d'un principe actif ou d'un mélange de principes actifs tels que décrits précédemment est déterminé par l'utilisation finale de la composition selon l'invention.

De manière préférée, la composition sèche stabilisée selon l'invention est un aliment, un complément alimentaire ou un médicament.

Les compléments alimentaires sont des produits destinés à être ingérés en compléments de l'alimentation courante, afin de pallier l'insuffisance réelle ou supposée des apports journaliers. Ils contribuent au bon fonctionnement de l'organisme et au bon rétablissement de l'équilibre des différentes fonctions qui l'animent quand celles-ci sont perturbées. Ces compléments alimentaires peuvent se présenter sous la forme de nutraceutiques qui sont des produits fabriqués à partir de produits alimentaires, mais rendu disponible sous forme de comprimé, de poudre, de potion ou d'autres formes médicinales habituellement non associés à des aliments.

La composition selon l'invention peut comporter en outre, dans sa partie pulvérulente et/ou dans les particules antimottantes, des adjuvants classiques pharmaceutiquement acceptables connus de l'Homme du métier ou des additifs alimentaires tels que des conservateurs, des anti-oxygènes, des acidifiants, des correcteurs d'acidité, des anti-moussants, des émulsifiants, des arômes, des agents appétents, des exhausteurs de goût, des agents moussants, des gélifiants, des humectants, des séquestrants, des stabilisants et des épaississants.

Bien entendu, l'Homme du métier veillera à choisir, de par ses connaissances, le ou les composés à ajouter aux compositions selon l'invention de telle manière que. les propriétés avantageuses selon la présente invention ne soient pas ou substantiellement pas altérées.

La composition selon l'invention résulte par exemple d'un simple mélange mécanique d'une partie pulvérulente contenant des principes actifs et de particules antimottantes tels que décrits ci-dessus. En pratique, elle comporte préférentiellement entre 40 et 95 % m/m (poids du poids total de la composition) de partie pulvérulente et entre 5 et 60 % m/m de particules antimottantes, et, plus préférentiellement, entre 60 et 90 % m/m de principes actifs sous forme pulvérulente et entre 10 et 40 % m/m de particules antimottantes.

Préférentiellement, la partie pulvérulente a une granulométrie moyenne comprise entre 20 et 100 µm et les particules antimottantes ont une granulométrie moyenne comprise entre 200 et 800 µm et au moins 2,5 fois supérieure à la granulométrie moyenne de la partie pulvérulente. De manière préférée, la granulométrie moyenne des particules antimottantes est au moins 4 fois supérieure à la granulométrie moyenne de la partie pulvérulente.

Par ailleurs, la composition selon l'invention est sèche, c'est-à-dire pas ou peu imprégnée de liquide et, en particulier d'eau. Elle présente préférentiellement une activité de l'eau inférieure à 0,4 et, plus préférentiellement, une activité de l'eau inférieure à 0,3.

En pratique, les compositions selon l'invention ont une date de péremption largement supérieure à la date de péremption que l'on aurait pu obtenir dans des compositions sèches pulvérulentes de l'état de la technique, conservées dans les mêmes conditions.

Le procédé de préparation d'une composition sèche stabilisée selon l'invention est **caractérisé en ce qu**'il comporte l'étape suivante de mélange de la partie pulvérulente de granulométrie moyenne dont la granulométrie moyenne est comprise entre 200 et 1 500 µm et est au moins 2,5 fois supérieure à la granulométrie moyenne dudit principe actif. Cette étape de mélange de la partie pulvérulente avec des particules antimottantes est effectuée selon les techniques habituelles connues.

De manière préférée, le procédé de préparation est réalisé dans des conditions d'humidité contrôlées assurant une activité de l'eau inférieure à 0,4 dans la composition obtenue.

L'invention concerne également l'utilisation de la composition selon l'invention comme aliment, comme complément alimentaire, ou comme médicament.

La composition selon l'invention se présente, sous une forme pulvérulente, utilisée en l'état ou destinée à être conditionnée sous différentes formes selon les applications ultérieures. Elle peut avantageusement servir à la fabrication de médicaments à usage humain ou vétérinaire, qui se présentent préférentiellement sous la forme de capsules ou gélules, éventuellement gastro-résistantes, à enveloppe dure ou molle.

Selon un mode de réalisation avantageux de l'invention, le médicament comprend avantageusement un excipient ou élément de charge de manière à former un prémélange sous la forme d'une poudre. Préférentiellement, l'excipient ou le mélange d'excipients utilisés pour former ledit prémélange sont identiques à ceux présents dans la partie pulvérulente de la composition selon l'invention.

La présente invention va maintenant être illustrée au moyen des exemples suivants.

### Exemple 1 : Composition pulvérulente contenant des extraits de plantes et sa préparation.

| **Constituants** | **Teneur (% m/m)** |
|---|---|
| Extraits de plantes absorbés sur maltodextrines (Passiflore, Valériane) | 69,00 |
| Sulfate de magnésium | 1,00 |
| Particules d'acide citrique anhydre de 300 à 700 µm | 30,00 |

La composition est préparée en mélangeant les principes actifs sous forme pulvérulente de granulométrie moyenne d'environ 50 µm (extraits de plantes absorbés sur maltodextrines) aux particules d'acide citrique anhydre.

### Exemple 2 : Composition pulvérulente contenant un mélange d'acides aminés et de vitamines.

| **Constituants** | **Teneur (% m/m)** |
|---|---|
| Mélange d'acides aminés et assimilés (lysine, méthionine, chlorure de choline) | 22,00 |
| Mélange de vitamines lipophiles sur support (vitamines A, D₃, E et K₃) | 33,00 |
| Mélange de vitamines du groupe B (feed grade (qualité alimentaire)) | 23,00 |
| (vitamines B₁, B₂, B₅, B₆, B₉, B₁₂) Vitamine C | 5,00 |
| Particules de mannitol | 17,00 |

La composition est préparée en mélangeant les principes actifs sous forme pulvérulente de granulométrie moyenne d'environ 50 µm (mélange d'acides aminés et de vitamines) aux particules de mannitol de 250 à 800 µm.

### Exemple 3 : Fabrication d'une composition pulvérulente contenant un mélange d'acides aminés et de vitamines

La composition est préparée en quatre phases :
- Phase 1 : le mannitol (76,70%), la crospovidone (3,10%) et la cellulose monocristalline (6,2%) sont granulés en présence d'une solution hydro-alcoolique de povidone (14%).
- Phase 2 : les particules de mannitol obtenues sont séchées puis calibrées pour que leur diamètre moyen soit compris entre 250 µm et 800 µm.
- Phase 3 : les particules de mannitol sont mélangées aux principes actifs sous forme pulvérulente.
- Phase 4 : la composition obtenue est ensuite conditionnée en sachets aluminisés soudés.

### Exemple 4 : Etude comparative de la prise en masse

La présente étude a pour but de comparer la prise en masse de la composition selon l'exemple 1 (composition A) avec une composition B comprenant les mêmes composants que la composition A mais dans laquelle les particules d'acide citrique ont été remplacées par de l'acide citrique en poudre d'une granulométrie faible (voisine de 25 µm).

Les concentrations des différents constituants des compositions A et B sont reprises ci-dessous :

| **Constituants** | **Composition A (% m/m)** | **Composition B (% m/m)** |
|---|---|---|
| Mélange d'extraits . | 69,00 | 69,00 |
| Sulfate de magnésium | 1,00 | 1,00 |
| Particules d'acide citrique | 30,00 | 0,00 |
| Acide citrique | 0,00 | 30,00 |

Afin de comparer la prise en masse des deux compositions, on place les différents pots des compositions A et B dans une étuve maintenue à une température de 40°C et en présence de 75% d'humidité. L'aspect physique des compositions est analysé chaque mois.

Les résultats expérimentaux sont repris dans le tableau ci-dessous :

| | Temps | | | |
|---|---|---|---|---|
| Composition | 0 | 1,5 mois | 3 mois | 6 mois |
| A | Poudre beige fluide présentant des grains blancs. | Idem T0 | Idem T0 Couleur beige légèrement plus soutenue. | Idem T0 Couleur beige légèrement plus foncée. |
| B | Poudre beige fluide. | Prise en masse, mais friable présentant des agglomérats | Prise en masse, formation d'un bloc, plus d'écoulement du pot, couleur beige foncé. | Prise en masse, formation d'un bloc, plus d'écoulement du pot, couleur marron. |

Ces résultats montrent que la composition A ne présente pas de prise en masse contrairement à la composition B. En conséquence, il semble que le remplacement de l'acide citrique en poudre d'une granulométrie voisine de 25 µm (composition B) par des particules d'acide citrique présentant une granulométrie comprise entre 300 µm et 700µm (composition A) a pour effet de supprimer la prise en masse et ainsi d'améliorer la stabilité de la composition selon l'invention.

En outre, compte tenu du fait qu'une semaine de conservation dans les conditions opératoires du présent exemple équivaut à environ 3 mois de conservation à 25°C, il est permis de conclure qu'une composition selon l'invention telle que la composition A, conservée à des températures de l'ordre de 25°C, devrait présenter une péremption au cours du temps nettement améliorée par rapport à des compositions substantiellement exemptes de particules.

## Revendications

1. Composition sèche stabilisée, comprenant au moins un principe actif, constituée d'une partie pulvérulente de granulométrie moyenne comprise entre 1 et 250 µm, **caractérisée en ce qu'**elle incorpore des particules antimottantes dont la granulométrie moyenne est comprise entre 200 et 1500 µm et est au moins 2,5 fois supérieure à la granulométrie moyenne de ladite partie pulvérulente.

2. Composition sèche stabilisée selon la revendication 1, **caractérisée en ce que** la granulométrie moyenne des particules antimottantes est au moins 4 fois supérieure à la granulométrie moyenne de ladite partie pulvérulente.

3. Composition sèche stabilisée selon l'une quelconque des revendications 1 ou 2, **caractérisée en ce qu'**elle comprend entre 5 et 60% m/m, préférentiellement, entre 10 et 40% m/m de particules antimottantes.

4. Composition sèche stabilisée selon l'une quelconque des revendications précédentes, **caractérisée en ce que** les particules antimottantes sont choisies parmi les sucres, les mélanges de saccharides, les acides organiques et leurs sels, les polyols, les sels inorganiques ou les particules creuses à base d'au moins un polymère ou copolymère acrylique ou méthacrylique, pris seuls ou en mélange.

5. Composition sèche stabilisée selon l'une quelconque des revendications précédentes, **caractérisée en ce que** les particules antimottantes sont poreuses et/ou creuses.

6. Composition sèche stabilisée selon l'une quelconque des revendications précédentes, **caractérisée en ce que** la partie pulvérulente est uniquement constituée de principes actifs.

7. Composition sèche stabilisée selon l'une quelconque des revendications précédentes, **caractérisée en ce que** les principes actifs sont compris, partiellement ou en totalité, dans les particules antimottantes.

8. Composition sèche stabilisée selon l'une quelconque des revendications précédentes, **caractérisée en ce que** les principes actifs sont choisis parmi les vitamines, les minéraux, les oligo-éléments, les acides aminés, les protéines, les enzymes, les facteurs de croissance, les poudres de plantes et leurs extraits, les inhibiteurs de croissance des insectes, les antibactériens, les antifongiques, les antiseptiques, les antibiotiques, les antibiomimétiques, les antiparasitaires et/ou les anti-infectieux de synthèse, pris seuls ou en mélange.

9. Composition sèche stabilisée selon l'une quelconque des revendications précédentes, **caractérisée en ce qu'**elle présente une activité de l'eau inférieure à 0,4.

10. Procédé de fabrication d'une composition sèche stabilisée, comprenant au moins un principe actif, constituée d'une partie pulvérulente de granulométrie moyenne comprise entre 1 et 250 µm, **caractérisé en ce qu'**il comporte l'étape de mélange de ladite partie pulvérulente avec des particules antimottantes dont la granulométrie moyenne est comprise entre 200 et 1 500 µm et est au moins 2,5 fois supérieure à la granulométrie moyenne de ladite partie pulvérulente.

11. procédé selon la revendication 10, **caractérisé en ce qu'**il est réalisé dans des conditions d'humidité contrôlées assurant une activité de l'eau inférieure à 0,4 dans la composition obtenue.

12. Utilisation de la composition sèche stabilisée selon l'une quelconque des revendications 1 à 9 pour la préparation d'un aliment.

13. Utilisation de la composition sèche stabilisée selon l'une quelconque des revendications 1 à 9 pour la préparation d'un complément alimentaire

14. Utilisation de la composition sèche stabilisée selon l'une quelconque des revendications 1 à 9 pour la préparation d'un médicament à usage humain ou vétérinaire.

15. Médicament **caractérisé en ce qu'**il comprend la composition sèche stabilisée selon l'une quelconque des revendications 1 à 9.

16. Médicament selon la revendication 15 **caractérisé en ce qu'**il se présente sous la forme de gélules ou de poudres.

## Claims

1. A stabilised dry composition, including at least one active principle, consisting of a powdery portion with an average particle size between 1 and 250 µm, **characterised in that** it incorporates anti-caking particles the average particle size of which is between 200 and 1500 µm and is at least 2.5 times higher than the average particle size of said powdery portion.

2. The stabilised dry composition according to claim 1, **characterised in that** the average particle size of the anti-caking particles is at least 4 times higher than the average particle size of said powdery portion.

3. The stabilised dry composition according to any of claims 1 or 2, **characterised in that** it includes between 5 and 60% w/w, preferably between 10 and 40% w/w, of anti-caking particles.

4. The stabilised dry composition according to any of the preceding claims, **characterised in that** the anti-caking particles are selected from sugars, saccharide mixtures, organic acids and salts thereof, polyols, inorganic salts or hollow particles based on at least one acrylic or methacrylic polymer or copolymer, taken alone or in admixture.

5. The stabilised dry composition according to any of the preceding claims, **characterised in that** the anti-caking particles are porous and/or hollow.

6. The stabilised dry composition according to any of the preceding claims, **characterised in that** the powdery portion only consists of active principles.

7. The stabilised dry composition according to any of the preceding claims, **characterised in that** the active principles are comprised, partially or totally, within the anti-caking particles.

8. The stabilised dry composition according to any of the preceding claims, **characterised in that** the active principles are selected from vitamins, minerals, trace elements, amino acids, proteins, enzymes, growth factors, plant powders and extracts thereof, insect growth inhibitors, antibacterials, antifungals, antiseptics, antibiotics, antibiomimetics, antiparasitics and/or synthetic antiinfectives, taken alone or in admixture.

9. The stabilised dry composition according to any of the preceding claims, **characterised in that** it has a water activity less than 0.4.

10. A method for making a stabilised dry composition, including at least one active principle, consisting of a powdery portion the average particle size of which is between 1 and 250 µm, **characterised in that** it includes the step of mixing said powdery portion with anti-caking particles the size of which is between 200 and 1500 µm and is at least 2.5 times higher than the average particle size of said powdery portion.

11. The method according to claim 10,
**characterised in that** it is carried out under controlled humidity conditions ensuring a water activity less than 0.4 in the composition obtained.

12. A use of the stabilised dry composition according to any of claims 1 to 9, for preparing food.

13. A use of the stabilised dry composition according to any of claims 1 to 9, for preparing a dietary supplement.

14. A use of the stabilised dry composition according to any of claims 1 to 9, for preparing a drug for human or veterinary use.

15. A drug **characterised in that** it includes the stabilised dry composition according to any of claims 1 to 9.

16. The drug according to claim 15, **characterised in that** it is in the form of gelatine capsules or powders.

## Patentansprüche

1. Stabilisierte trockene Zusammensetzung, umfassend mindestens einen Wirkstoff, bestehend aus einem pulverförmigen Teil mit einer mittleren Granulometrie zwischen 1 und 250 µm, **dadurch gekennzeichnet, dass** sie Antianbackpartikel umfasst, deren mittlere Granulometrie zwischen 200 und 1500 µm liegt und mindestens 2,5 Mal höher ist als die mittlere Granulometrie des pulverförmigen Teils.

2. Stabilisierte trockene Zusammensetzung nach Anspruch 1, **dadurch gekennzeichnet, dass** die mittlere Granulometrie der Antianbackpartikel mindestens 4 Mal höher als die mittlere Granulometrie des pulverförmigen Teils ist.

3. Stabilisierte trockene Zusammensetzung nach einem der Ansprüche 1 oder 2, **dadurch gekennzeichnet, dass** sie zwischen 5 und 60% m/m, vorzugsweise zwischen 10 und 40% m/m Antianbackpartikel umfasst.

4. Stabilisierte trockene Zusammensetzung nach einem der vorhergehenden Ansprüche, **dadurch gekennzeichnet, dass** die Antianbackpartikel unter den Zuckern, den Mischungen von Sacchariden, den organischen Säuren und ihren Salzen, den Polyolen, den anorganischen Salzen oder den Hohlpartikeln auf der Grundlage mindestens eines Akryl- oder Metakrylpolymers oder -copolymers, alleine oder in einer Mischung, gewählt werden.

5. Stabilisierte trockene Zusammensetzung nach einem der vorhergehenden Ansprüche, **dadurch gekennzeichnet, dass** die Antianbackpartikel porös und/oder hohl sind.

6. Stabilisierte trockene Zusammensetzung nach einem der vorhergehenden Ansprüche, **dadurch gekennzeichnet, dass** der pulverförmige Teil ausschließlich aus Wirkstoffen besteht.

7. Stabilisierte trockene Zusammensetzung nach einem der vorhergehenden Ansprüche, **dadurch gekennzeichnet, dass** die Wirkstoffe vollständig oder teilweise in den Antianpackpartikeln enthalten sind.

8. Stabilisierte trockene Zusammenfassung nach einem der vorhergehenden Ansprüche, **dadurch gekennzeichnet, dass** die Wirkstoffe unter den Vitaminen, den Mineralien, den Oligoelementen, den Aminosäuren, den Proteinen, den Enzymen, den Wachstumsfaktoren, den Pflanzenpulvern und ihren Extrakten, den Wachstumshemmern von Insekten, den antibakteriellen, fungiziden, antiseptischen, antibiotischen, antibiomimetischen, antiparasitären und/oder den Antiinfektionsmitteln, alleine oder in Mischung, gewählt werden.

9. Stabilisierte trockene Zusammensetzung nach deinem der vorhergehenden Ansprüche, **dadurch gekennzeichnet, dass** sie eine Wasseraktivität von weniger als 0,4 aufweist.

10. Herstellungsverfahren einer stabilisierten trockenen Zusammensetzung, umfassend mindestens einen Wirkstoff, bestehend aus einem pulverförmigen Teil mit einer mittleren Granulometrie zwischen 1 und 250 µm, **dadurch gekennzeichnet, dass** es den Schritt des Mischens des pulverförmigen Teils mit den Antianbackpartikeln umfasst, deren mittlere Granulometrie zwischen 200 und 1500 µm liegt und mindestens 2,5 Mal höher als die mittlere Granulometrie des pulverförmigen Teils ist.

11. Verfahren nach Anspruch 10, **dadurch gekennzeichnet, dass** es unter kontrollierten Feuchtigkeitsbedingungen durchgeführt wird, die eine Wasseraktivität von weniger als 0,4 in der erhaltenen Zusammensetzung sicherstellen.

12. Verwendung der stabilisierten trockenen Zusammensetzung nach einem der Ansprüche 1 bis 9 für die Zubereitung eines Nahrungsmittels.

13. Verwendung der stabilisierten trockenen Zusammensetzung nach einem der Ansprüche 1 bis 9 für die Zubereitung eines Nahrungsmittelzusatzes.

14. Verwendung der stabilisierten trockenen Zusammensetzung nach einem der Ansprüche 1 bis 9 für die Zubereitung eines Medikamentes zur Verwendung beim Menschen oder bei Tieren.

15. Medikament, **dadurch gekennzeichnet, dass** es die stabilisierte trockene Zusammensetzung nach einem der Ansprüche 1 bis 9 umfasst.

16. Medikament nach Anspruch 15, **dadurch gekennzeichnet, dass** es in der Form von Kapseln oder von Pulvern vorliegt.
